# EUROPÄISCHE PATENTANMELDUNG

(11) **EP 3 305 830 A1**
(43) Veröffentlichungstag der Anmeldung: **11.04.2018**
(21) Anmeldenummer: 16192527.6
(22) Anmeldetag: 06.10.2016
(51) Int. Cl.: C08G 73/06, C09J 179/04

(54) **CYANATESTER-BASIERTER KLEBSTOFF UND VERFAHREN FÜR DIE HERSTELLUNG EINES CYANATESTER-BASIERTEN KLEBSTOFFS**

(71) Anmelder: nolax AG, 6203 Sempach Station (CH)
(72) Erfinder: Läpple, Markus, 6210 Sursee (CH); Zimmermann, Erika, 6374 Buochs (CH); Buser, Stephan, 6210 Sursee (CH); Müller, Alfredo, 8008 Zürich (CH)
(74) Vertreter: Hepp Wenger Ryffel AG

(57) **Zusammenfassung**

Die Erfindung betrifft einen Cyanatester-basierter Klebstoff umfassend eine Komponente A, welche mindestens einen Cyanatester mit mindestens zwei OCN-Gruppen aufweist, und eine Komponente B, welche mindestens einen Katalysator für die Trimerisierungsreaktion von OCN-Gruppen zu einem Triazinring aufweist. Dabei ist der Katalysator auf einem Träger, insbesondere einer pyrogenen Kieselsäure, reversibel zurückgehalten.

## Beschreibung

Die Erfindung betrifft einen Cyanatester-basierten Klebstoff, ein Verfahren für die Herstellung eines Cyanatester-basierten Klebstoffs sowie die Verwendung eines Cyanatester-basierten Klebstoffs für die Beschichtung von Substraten.

Für die Laminierung von Hochtemperatur-Isolationsmaterialien wie beispielsweise einem Glasfasermatte/Aluminiumfolie-Verbund im Motorraum oder der Auspuffanlagen eines Fahrzeugs mit Langzeitbeständigkeiten sowie Hochtemperaturbeständigkeiten (> 300°C) gibt es heute nur eine eingeschränkte Klebstoffauswahl auf dem Markt. Neben anorganischen Systemen, welche meist auf Silikat basieren, gibt es noch Polyimid- und Cyanatester-basierte Klebstoffsysteme sowie Silikonklebstoffe. Cyanatester-Klebstoffe werden heute meist hergestellt, indem ein Katalysator für die Polymerisationsreaktion des Cyanatesters unmittelbar vor der Verarbeitung eingemischt, die Klebstoffzusammensetzung sofort aufgetragen, laminiert und anschliessend thermisch beispielsweise bei 150 bis 250°C vernetzt wird. Das Klebstoffsystem aus Cyanatester und Katalysator ist in der Regel nicht lagerfähig bei Raumtemperatur. Mit Cyanatester-vorbeschichtete Halbzeuge müssen daher bei sehr niedrigen Temperaturen beispielsweise bei -21°C gelagert werden.

Prinzipiell vernetzen drei Cyanatgruppen von multifunktionalen Cyanatestern in Anwesenheit eines geeigneten Katalysators und durch Wärmeeintrag zu einem Triazinring, wodurch ein Cyanatester-basierter Klebstoff unter Bildung eines dreidimensionalen Netzwerks aushärtet.

Aus EP 1 265 947 B1 sind schnellhärtende Polymere aus Polycyanaten und Polycanat/Epoxid-Kombinationen mit aminischen Härtern als Katalysatoren bekannt. Die Amin-Katalysatoren liegen in der Klebstoffzusammensetzung verkapselt vor, und werden bspw. durch Schmelzen der Kapseln bei erhöhten Temperaturen freigesetzt, so dass der Härtungsprozess startet. Nachteilig daran sind die aufwendige Bereitstellung des Katalysators in Kapselform sowie lokalen Überhitzungen während der Polymerisierungsreaktion.

Aus WO 2012/139940 A1 sind Polyurethan-Zusammensetzungen mit komplexierten Metall-Katalysatoren bekannt, wobei während einer Induktionsphase die katalytische Reaktivität noch vermindert ist und nach dieser Induktionsperiode aber hohe Reaktivität und katalytische Wirkung erreicht werden, um eine schnelle Vernetzung des zu vernetzenden Systems zu erzeugen. Die Komplexierung erfolgt über retardierende Substanzen. Nachteilig daran ist, dass die beschriebenen Polyurethan-Zusammensetzungen auf komplexierbare Katalysatorklassen beschränkt sind.

Im Allgemeinen ist bei Cynatester-Beschichtungen das Problem bekannt, dass der Katalysator lokal überkonzentriert vorliegt, was zu einem unkontrollierten exothermen Reaktionsverlauf führen kann. Des Weiteren lassen sich herkömmliche Katalysatoren nur mit hohem Aufwand homogen einarbeiten.

Aufgabe der Erfindung ist es, die Nachteile des Standes der Technik zu überwinden. Insbesondere sind es Aufgaben der Erfindung einen lagerstabilen Cyanatester-basierten Klebstoff zur Verfügung zu stellen sowie ein Verfahren zur Herstellung eines lagerstabilen Cyanatester-basierten Klebstoffs zur Verfügung zu stellen. Des Weiteren sollen lokale Überkonzentrationen des Katalysators vermieden werden. Diese Aufgaben werden mit den unabhängigen Ansprüchen 1 und 5 gelöst.

Die Erfindung betrifft einen Cyanatester-basierten Klebstoff, umfassend eine Komponente A und eine Komponente B. Die Komponente A weist mindestens einen Cyanatester mit mindestens zwei OCN-Gruppen auf. Die Komponente B weist mindestens einen Katalysator für die Trimerisierungsreaktion von OCN-Gruppen zu einem Triazinring auf. Dabei ist der Katalysator auf einem Träger, insbesondere einer pyrogenen Kieselsäure, reversibel zurückgehalten. Unter "reversibel zurückgehalten" wird hier und im Folgenden verstanden, dass der Katalysator auf der Oberfläche des Trägers anhaftet bzw. mit der Oberfläche des Trägers in Kontakt steht. Dies kann insbesondere durch physikalische Adsorption oder chemische Adsorption erfolgen. Auf diese Weise wird der Katalysator in der Zusammensetzung gleichmässig verteilt und eine lokale Überkonzentration bei der Freisetzung des Katalysators vermieden. Der Katalysator kann so deutlich besser eingemischt werden. Aufgrund des an dem Träger reversibel zurückgehaltenen Katalysators wird die Lagerstabilität der Zusammensetzung zudem um ein Vielfaches erhöht.

Da der Cyanatester-basierten Klebstoff vorteilhafterweise latent reaktiv ist, kann ein Substrat mit dem Cyanatester-basierten Klebstoff beschichtet werden, ohne dass der Cyanatester-basierte Klebstoff aushärtet, bzw. sich seine Viskosität mit der Zeit signifikant erhöht. Das beschichtete Substrat kann zwischengelagert werden bevor in weiteren Arbeitsschritten bspw. der Cyanatester-basierte Klebstoff ausgehärtet und/oder weitere Bauelemente an das beschichtete Substrat angeordnet werden. Bei der Polymerisationsreaktion, welche stark exotherm ist, entstehen keine nachteiligen lokalen Überhitzungen aufgrund lokaler heterogener Einmischung des Katalysators. Es sind des Weiteren keine wie aus dem Stand der Technik bekannten retardierenden Substanzen notwendig, welche beispielsweise den Katalysator komplexieren und so die Reaktivität der Zusammensetzung herabsetzen. Jedenfalls wird eine wesentlich harmonischer verlaufende Abreaktion beobachtet als mit verkapselten oder komplexierten Katalysatoren. Als besonders vorteilhaft hat sich erwiesen, dass ein erfindungsgemässer Cyanatester-basierter Klebstoff bei einer Wärmelagerung bei 60°C eine Stabilität von mehreren Tagen und bei RT eine Stabilität von > 3 Monaten aufweist ohne einen nennenswerten Anstieg der Viskosität. Bei bekannten Cyanatester-basierten Zusammensetzungen wird ein Anstieg der Viskosität während der Lagerung bei Raumtemperatur (ca. 20 °C) aufgrund der beginnenden Polymersierungsreaktion registriert, was nachteilig ist.

Der mindestens eine Katalysator kann im nicht-komplexierten Zustand vorliegen. Derart muss keine komplexierende Komponente in den Cyanatester-basierten Klebstoff eingearbeitet oder ein Katalysator vorgängig mit einer komplexierenden Komponente in Kontakt gebracht werden.

Der mindestens eine Katalysator kann bevorzugt Zinnnoctoat sein. Zinnoctoat, welches an einem Träger, insbesondere pyrogener Kieselsäure, reversibel zurückgehalten ist, hat sich als bevorzugter Katalysator für die Trimerisierungsreaktion von OCN-Gruppen zu einem Triazinring und damit die Aushärtung des Cyanatester-basierten Klebstoffs herausgestellt. Zinnoctoat eignet sich besonders gut als Katalysator, da es im Vergleich zu Fe(III)- und Co-Katalysatoren inaktiv bei niedrigen Temperaturen ist, und sich des Weiteren vorteilhaft durch eine geringe Toxizität und hohe Verfügbarkeit auszeichnet. Des Weiteren hat sich gezeigt, dass die Viskosität mit Zinnoctoat deutlich weniger ansteigt als mit den üblichen Katalysatoren wie bspw. Fe(III)Acetylacetonat oder Mn(II)Acetylacetonat.

Die Komponente B kann einen oder mehrere Katalysatoren für die Trimerisierungsreaktion von OCN-Gruppen zu einem Triazinring und damit die Aushärtung des Cyanatester-basierten Klebstoffs aufweisen. Der Katalysator oder die Katalysatoren können anteilig 0,001 bis 10 Gew.-%, besonders bevorzugt 0,01 bis 5 Gew.-%, besonders bevorzugt 0,1 bis 1 Gew.-%, bezogen auf den Cyanatester ausmachen. Die vorgenannten Gew.-% beziehen sich auf die Gesamtzusammensetzung.

Der Cyanatester-basierte Klebstoff kann mindestens einen Füllstoff zu einem Anteil von mindesten 5 Gew.-% bevorzugt mindestens 25 Gew.-%, besonders bevorzugt 30 Gew.-% enthalten. Als Füllstoffe eignen sich anorganische Füllstoffe wie beispielsweise Calciumcarbonat (Kreide), Kaolin, Montmorrilonit (Bentonit), Wollastonit, pyrogene Kieselsäure, Glasmehl, Altglasmehl, Glashohlkugeln, Pigmente, Fasern aus Glas oder Basalt, Talk oder Farbmittel. Als Füllstoffe eignen sich ebenso organische Füllstoffe wie beispielsweise Silikone, Kautschuke oder bekannte Schlagzähigkeitmodifizierer. Es kann ein einzelner Füllstoff oder mehrere Füllstoffe eigesetzt werden. Der Füllstoffgehalt kann 25 bis 30 Gew.-% betragen. Der Zusatz von flammhemmenden Additiven ist nicht notwendig, da der ausreagierte Klebstoff nur sehr schwer entzündlich ist. Prinzipiell ausgeschlossen ist der Zusatz von flammhemmenden Additiven jedoch nicht.

Ein weiterer Aspekt der Erfindung betrifft ein Verfahren zur Herstellung eines Cyanatester-basierten Klebstoffes, insbesondere wie vorgängig erläutert. Das Verfahren umfasst den Schritt a) Inkontaktbringen, insbesondere Mischen, einer Komponente A und einer Komponente B in einer Lösung. Dabei weist die Komponenten A mindestens einen Cyanatester mit mindestens zwei OCN-Gruppen auf. Die Komponente B weist mindestens einen Katalysator, insbesondere Zinnoctoat, für die Trimerisierungsreaktion von OCN-Gruppen zu einem Triazinring auf. Dabei ist der Katalysator auf einem Träger, insbesondere einer pyrogenen Kieselsäure, reversibel zurückgehalten. Optional weist das Verfahren den Schritt b) auf, wobei die Zusammensetzung aus a) im Vakuum entgast wird. Auf diese Weise wird ein Verfahren für die Herstellung eines Cyanatester-basierten Klebstoffes bereitgestellt, welches einfach und reproduzierbar durchführbar ist. Des Weiteren wird mit dem Verfahren ein Cyanatester-basierten Klebstoff bereitgestellt, welcher die vorgängig genannten Vorteile aufweist.

Das Inkontaktbringen, insbesondere Mischen, in Schritt a) kann bei 20 bis 100°C, bevorzugt bei 30 bis 70°C, besonders bevorzugt bei mindestens 60°C, für 1 bis 10 min, bevorzugt 2 bis 8 min, besonders bevorzugt 4 bis 6 min erfolgen. Derart werden die einzelnen Komponenten der Zusammensetzung besonders gut verteilt und lokale Überkonzentrationen einzelner Komponenten ausgeschlossen.

Die Komponente A und/oder die Komponente B können als Lösung/en im Schritt a) des Verfahrens bereitgestellt werden. Dies bedeutet, dass entweder die Komponente A in Lösung vorgelegt und Komponenten B als Feststoff in die Lösung der Komponente A eingebracht wird oder dass die Komponente B in Lösung vorgelegt und Komponenten A als Feststoff in die Lösung der Komponente B eingebracht wird. Auf diese Weise ist frei wählbar, ob eine der beiden Komponenten oder beide Komponenten bereits in Lösung vorliegen. Entsprechend kann der Anwender situationsbedingt wählen.

Die Komponenten A und B können als Feststoffe in Schritt a) bereitgestellt werden und in einem Lösungsmittel aufgenommen werden. Geeignete Lösungsmittel sind dem Fachmann bekannt. Bevorzugte Lösungsmittel sind aprotische Lösungsmittel, die einen Siedepunkt von kleiner 200 °C haben. Die Lösungsmittel lassen sich derart vorteilhaft während dem Aushärtungsschritt entfernen. Eine nicht abschliessende Liste geeigneter Lösungsmittel umfasst Aceton, Dichlormethan, Tetrahydrofuran, Diethylenglycoldimethyl- oder diethylether, Diethylenglycolmonobutyletheracetat, Ethylmethylketon, N-Methylpyrrolydon, Acetonitril, Dimethylformamid (DMF), Dimethylsulfoxid (DMSO), Dimethylcarbonat, Tetrahydrofuran (THF), Dichlormethan, Ethylenchlorid, Ethylacetat. Auf diese Weise können die Komponenten A und B als Feststoffe vorgemischt und in einem Lösungsmittel aufgenommen werden, was die Handhabbarkeit verbessert.

Zum Aufbringen des Katalysators, insbesondere des Zinnoctoats, an dem Träger, insbesondere der pyrogenen Kieselsäure, kann der Katalysator in einem Lösungsmittel, bevorzugt Aceton, gelöst werden. Der Träger, insbesondere die pyrogene Kieselsäure kann in das Lösungsmittel, welches den Katalysator aufweist, zugegeben werden und so mit dem Katalysator in Kontakt gebracht werden. Die Zusammensetzung aus Katalysator und Träger kann gemischt, insbesondere dispergiert, werden. Anschliessend kann das Lösungsmittel, insbesondere mittels Verdampfen, entfernt werden. Optional kann der mit dem daran reversibel zurückgehaltenen Katalysator nach dem Entfernen des Lösungsmittels gemahlen werden. Auf diese Weise lässt sich ein Katalysator besonders einfach an einem Träger, insbesondere einer pyrogenen Kieselsäure, aufbringen, so dass der Katalysator an dem Träger reversibel zurückgehalten wird, und kann in dem erfindungsgemässen Verfahren zur Herstellung einer Cyanatester-basierten Klebstoffs bereitgestellt werden.

Die Erfindung betrifft weiter die Verwendung von Zinnoctoat als Katalysator für die Trimerisierungsreaktion von OCN-Gruppen zu einem Triazinring eines Cyanatester-basierten Klebstoffes. Dabei ist das Zinnoctoat an einem Träger, insbesondere einer pyrogenen Kieselsäure, reversibel zurückgehalten. Die vorgängig erwähnten Vorteile für Zinnoctoat als Katalysator gelten selbstverständlich gleichermassen für die Verwendung von Zinnoctoat als Katalysator für die für die Trimerisierungsreaktion von OCN-Gruppen zu einem Triazinring eines Cyanatester-basierten Klebstoffes.

Ein weiterer Aspekt der Erfindung ist die Verwendung eines Cyanatester-basierten Klebstoffs wie vorgängig erläutert für die Beschichtung eines Substrats. Dabei weist der Cyanatester-basierte Klebstoff die vorgängig erwähnten Charakteristika sowie die vorteilhaften Eigenschaften auf.

Die Erfindung betrifft weiter ein Substrat mit einer Beschichtung mit einem Cyanatester-basierten Klebstoff wie vorgängig erläutert. Ein derart beschichtetes Substrat kann auf unterschiedlichste Elemente, insbesondere Bauteile, aufgebracht, insbesondere angebracht, werden.

Weiter betrifft die Erfindung die Verwendung eines Substrats, welches mit einem Cyanatester-basierten Klebstoff wie vorgängig erläutert beschichtet ist, für die Isolierung eines Bauteils im Bereich Automobilbau/-industrie, Hausbau, Schiffsbau, Ofenbau, Hochofenbau, Chemiereaktoren, Flugzeugbau/-industrie, Luft- und Raumfahrt, Blockheizkraftwerke, Zementofen, Kehrricht-/Abfallverbrennungsofen. Dazu wird beispielsweise das Substrat mit einem Cyanatester-basierten Klebstoff beschichtet, ein Bauteil auf die Beschichtung aufgebracht und die Beschichtung mittels Wärmeeintrag polymerisiert bzw. gehärtet. Daraus resultiert ein Bauteil, welches eine Isolierung aus dem Substrat aufweist.

Ein weiterer Aspekt der Erfindung betrifft ein Verfahren zur Beschichtung eines Bauteils mit einem Cyanatester-basierten Klebstoff wie vorgängig erläutert. Das Verfahren umfasst die Schritte a) Aufbringen eines Cyanatester-basierten Klebstoffes auf ein Substrat, b) Aufbringen des Bauteils auf den auf das Substrat aufgebrachten Cyanatester-basierten Klebstoff, und c) Vernetzen des Cyanatester-basierten Klebstoffes bei einer Temperatur von > 100°C, bevorzugt bei 220 °C, für 1 bis 30 min, bevorzugt 5 bis 10 min. Auf diese Weisen werden Bauteile mit einer Beschichtung, insbesondere einer Wärmeisolierung für hohe Temperaturen, besonders einfach hergestellt.

Das Vernetzen bzw. Polymerisieren des Cyanatester-basierten Klebstoffes kann unter Ausschluss von Luftfeuchtigkeit erfolgen. Dies kann beispielsweise in einer MEYER Bandpresse erfolgen. Die Bandpresse hat den Vorteil, dass eine kontrollierte Temperaturführung möglich ist. Des Weiteren können Substrate, insbesondere bahnförmiges Material über die gesamte Breite, mit dem latent reaktiven Cyanatester-basierten Klebstoff effizient vorbeschichtet werden. Das vorbeschichtete Substrat kann ohne Kühlung gelagert, transportiert und/oder konfektioniert werden. Erst für die endgültige Aushärtung kann die Reaktion durch Erhöhen der Temperatur ausgelöst werden.

Das Substrat und/oder das Bauteil können vorbehandelt sein. Mögliche Vorbehandlungen sind dabei insbesondere Inaktivierung der Oberfläche mittels chemischer und/oder physikalischer Reinigung, insbesondere Corona-Vorbehandlung, Plasmabehandlung. Textil kann insbesondere mit Beflammung vorbehandelt werden. Folien, insbesondere Alufolien, können insbesondere mit einer Antifog-spray-Beschichtung vorbehandelt werden. Auf diese Weise wird eine besonders gute Haftung zwischen dem Substrat und dem Bauteil erzielt.

Ein weiterer Aspekt der Erfindung betrifft ein System im Sinne eines kit of parts. Das System umfasst (i) mindestens einen Cyanatester aufweisend mindestens zwei OCN-Gruppen, (ii) mindestens zwei unterschiedliche Katalysatoren für die Trimerisierungsreaktion von OCN-Gruppen zu einem Triazinring, welche an einem Träger, insbesondere einer pyrogenen Kieselsäure, reversibel zurückgehalten sind, sowie (iii) optional Anwendungshinweise für ein Verfahren zur Beschichtung eines Bauteils wie vorgängig erläutert.

### MULTIFUNKTIONALE CYANATESTER

Bevorzugte Cyanatester aufweisend mindestens zwei OCN-Gruppen basieren auf Novolaken, beispielsweise bekannt unter den Handelsnamen Huntsman Cyanatester Typ AroCy XU 371 oder Primaset™ Cyanate Esters von Lonza.

Novolake sind säurekatalytisch hergestellte Polykondensationsprodukte aus Formaldehyd und Phenolen und sind der Gruppe der Phenolharze zuzuordnen (vgl. Chemie Lexikon, Römpp (Hrsg.), 9. Aufl. 1991, Bd. 4, Eintrag zum Stichwort "Novolake").

### METALLISCHE KATALYSATOREN

Es handelt sich dabei um Verbindungen, die ein Metallatom zusammen mit organischen Resten aufweisen. Beispiele sind Metalle, wie Zinn, Titan, Zink, Blei, Wismut, Eisen, Kobalt, Nickel, Calcium, Barium, Mangan, Vanadium, Zirkon, und/oder Aluminium. Diese können beispielsweise als Carbonsäuresalz, Chelat, wie beispielsweise Acetylacetonat, Hydroxid, Alkoholat, Phenolat, Oxid vorliegen, es sind auch gemischte Gruppen in dem Metallkatalysator bekannt. Es handelt sich dabei insbesondere um Verbindungen von Sn, Fe, Ti, Al, Bi oder Zr als Carbonsäuresalz, Oxid, Hydroxid oder Acetylacetonat. Ein bevorzugter metallischer Katalysator ist Zinnoctoat. Zinnoctoat ist auch als Zinnethylhexanoat bzw. Zinn(II)-2-ethylhexanoat bekannt. Die Menge kann 0,001 bis 10 Gew.-% bezogen auf die gesamte Zusammensetzung betragen. Bevorzugte Mengen sind 0,01 bis 5 Gew.-%, insbesondere 0,1 bis 1 Gew.-%.

### NICHTMETALLISCHE KATALYSATOREN

Bevorzugte nichtmetallische Katalysatoren sind substituierte Harnstoffe, insbesondere Diphenylurea (von Sigma Aldrich), Imidazole, insbesondere Dimethylimidazol oder 2,4-diamino-6-[2'-methylimidazolyl-(1')]-ethyl-s-triazine (Curezol 2 MZ Azine von Air Products), oder Tetraalkylammoniumcarboxylate, insbesondere Tetraethylammoniumbenzoate.

### TRÄGERSUBSTANZEN

Als Trägersubstanzen sind bevorzugt pyrogene Kieselsäure, beispielsweise Aerosil 200 von Evonik, oder pyrogene Metalloxide, beispielsweise Aeroxide oder Aluminiumoxide. Poröse Träger können Zeolithe sowie gefällte Kieselsäuren (precipitated silica) z.B. Sipernat von Evonik sein.

Bevorzugt weisen die Träger eine BET-Oberfläche im Bereich von 5 bis 400 g/m2, besonders bevorzugt im Bereich von 10 bis 200 g/m2 auf.

### BEISPIELE

Nachfolgend werden Aspekte des erfindungsgemässen Cyanatester-basierten Klebstoffs beziehungsweise des Verfahrens zur Herstellung des Cyanatester-basierten Klebstoffs anhand von Ausführungsbeispielen näher erläutert.

Die verwendeten Materialien sind nachfolgende aufgelistet:

| Handelsname | Material | Hersteller |
|---|---|---|
| Primaset PT-30 | Novolac-basierter Cyanatester, 100% Feststoffgehalt | Lonza |
| Aerosil 200 | pyrogene Kieselsäure | Evonik |
| WorleeAdd ST-70 | Flüssiges Zinnoctoat auf einem amorphen Silikatpulver als Trägersubstanz | Worlee-Chemie |

### Vergleichsbeispiel

Das Vergleichsbeispiel betrifft das Einmischen von Zinnoctoat ohne Adsorptionsschritt an einer pyrogenen Kieselsäure in einen Cyanatester-basierten Klebstoff. Dazu wurden 245 g Novolac-basierter Cyanatester mit 100% Feststoffgehalt (Primaset PT-30, Lonza) bei 60°C vorgewärmt, 0.245 g Zinnoctoat (Borchi Kat 28) und 9.8 g pyrogene Kieselsäure (Aerosil 200, Evonik) zugegeben und mit einem Labormischer bei 750 UpM (Umdrehungen pro Minute) gemischt. Die Mischung wurde bei 60°C gelagert.

### Ausführungsbeispiel

Das Ausführungsbeispiel betrifft das Einmischen von Zinnoctoat, welches reversibel an einer pyrogenen Kieselsäure zurückgehalten ist (WorleeAdd ST-70), in einen Cyanatester-basierten Klebstoff. Dazu wurden zunächst 245 g Primaset PT-30 im Ofen bei 60°C vorgewärmt. Anschliessend wurden 0.355 g Zinnoctoat, welches reversibel an einer pyrogenen Kieselsäure zurückgehalten ist (WorleeAdd ST-70), sowie 9.7 g pyrogene Kieselsäure (Aerosil 200, Evonik) zugegeben und in einem Labormischer bei 750 UpM dispergiert. Die Mischung wurde bei 60°C gelagert.

### DSC-Messungen (Netzsch DSC 204 F1 Phoenix mit Autosampler und Intracooler ETK100/A)

| | Area J/g | Reaktionsbereich [°C] | Reaktionspeak [°C] |
|---|---|---|---|
| **Vergleichsbeispiel** | 578 | 159 bis 330 | 238 / 261 |
| **Ausführungsbeispiel** | 600 | 170 bis 329 | 264 |

Die DSC-Messungen wurden in einem kalt verschweissten 25 µl Aluminium-Tiegel mit Aluminimum-Deckel durchgeführt. 9 bis 11 mg einer Klebstoffprobe gemäss Vergleichsbeispiel und Ausführungsbeispiel wurde eingewogen. Die Messung wurde bei 25°C bis 400°C mit 10 °C/min durchgeführt mit synthetischer Luftspülung (40 ml/min), gefolgt von einem isothermem Schritt bei 400°C für 5h mit synthetischer Luftspülung (40 ml/min).

### Brookfield-Viskositätsmessungen (Brookfiekd RVDVI+)

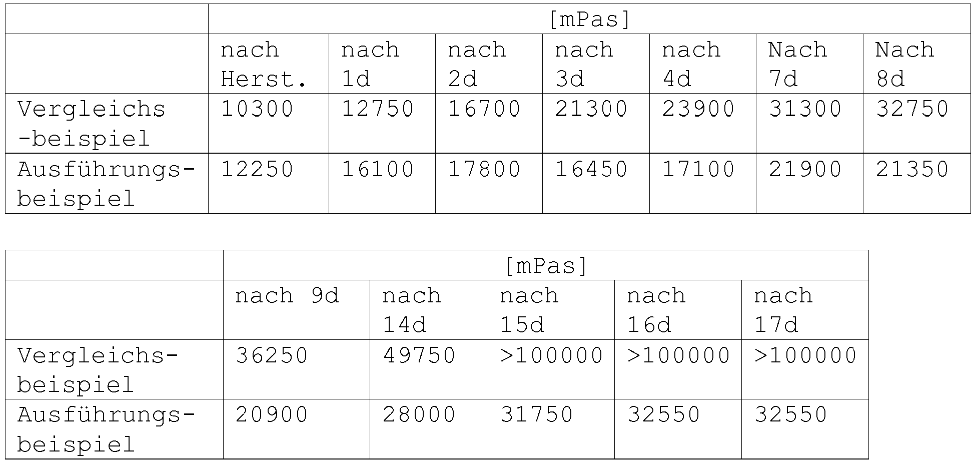

Die Klebstoffe gemäss Vergleichsbeispiel und Ausführungsbeispiel wurden für eine Dauer wie in der oben stehenden Tabelle angegeben bei 60 °C vor der Viskositätsmessung gelagert.

Nach 7 Monaten Lagerung bei Raumtemperatur (ca. 20 bis 23 °C) wiesen das Vergleichsbeispiel eine Brookfield-Viskosität von >100000 mPas und das Ausführungsbeispiel eine Brookfield-Viskosität von 23800 mPas auf.

Die Brookfield-Viskositätsmessungen wurden nach der Methode B der "ASTM D1084 - 97 (Reapproved 2005)" (Standard Test Methods for Viscosity of Adhesives) durchgeführt. Dabei wurde mit Spindel #6 und 20 rpm bei 60 °C gemessen.

Das erfindungsgemässe Ausführungsbeispiel weist im Vergleich zum Vergleichsbeispiel eine signifikant verbesserte Lagerstabilität auf. Während des Cyanatester-basierte Klebstoff gemäss dem Vergleichsbeispiel bereits nach 3 Tagen Lagerung eine signifikant höhere Viskosität aufweist und die Viskosität nach 15 Tagen nicht mehr messbar ist, weist der Cyanatester-basierte Klebstoff des Ausführungsbeispiels vergleichsweise niedrige Viskositätswerte über mindestens 17 Tage auf. Der Klebstoff gemäss dem Ausführungsbeispiel lässt sich entsprechend deutlich länger nach der Herstellung verarbeiten.

Die so hergestellten Cyanatester-basierten Klebstoffe lassen sich mit handelsüblichen Hotmelt Beschichtungsanlagen bei 60 bis 90°C auf Gewebe (vorzugsweise Glas) oder Aluminiumfolie auftragen. Derart sind die Gewebe mit einem unvernetzten Cyanatester-basierten Klebstoff beschichtet, d.h. es erfolgt keine Vernetzung während der Beschichtung der Gewebe. Die typischen Auftragsgewichte liegen zwischen 30 und 70 g/m². Zum Einsatz kommen Gravurwalze (Cavitec, Lacom), Rasterwalze positive oder Screen (System Nordson, Cavitec, J. Zimmer).

## Patentansprüche

1. Cyanatester-basierter Klebstoff umfassend eine
- Komponente A, welche mindestens einen Cyanatester mit mindestens zwei OCN-Gruppen aufweist, und
- eine Komponente B, welche mindestens einen Katalysator für die Trimerisierungsreaktion von OCN-Gruppen zu einem Triazinring aufweist,
wobei der Katalysator auf einem Träger, insbesondere einer pyrogenen Kieselsäure, reversibel zurückgehalten ist.

2. Cyanatester-basierter Klebstoff gemäss Anspruch 1, wobei der mindestens eine Katalysator im nicht-komplexierten Zustand vorliegt.

3. Cyanatester-basierter Klebstoff gemäss Anspruch 1, wobei der mindestens eine Katalysator Zinnoctoat ist.

4. Verfahren zur Herstellung eines Cyanatester-basierten Klebstoffes, insbesondere nach einem der Ansprüche 1 bis 3, umfassend die Schritte
a) Inkontaktbringen, insbesondere Mischen, einer Komponente A, welche mindestens einen Cyanatester mit mindestens zwei OCN-Gruppen aufweist, und einer Komponente B, welche mindestens einen Katalysator, insbesondere Zinnoctoat, für die Trimerisierungsreaktion von OCN-Gruppen zu einem Triazinring aufweist, in einer Lösung, wobei der Katalysator auf einem Träger, insbesondere einer pyrogenen Kieselsäure, reversibel zurückgehalten ist,
b) optional Entgasen der Zusammensetzung aus a) im Vakuum.

5. Verfahren gemäss Anspruch 4, wobei das Inkontaktbringen, insbesondere Mischen, in Schritt a) bei 20 bis 100°C, bevorzugt bei 30 bis 70°C, besonders bevorzugt bei mindestens 60°C, für 1 bis 10 min, bevorzugt 2 bis 8 min, besonders bevorzugt 4 bis 6 min erfolgt.

6. Verfahren gemäss Anspruch 4 oder 5, wobei die Komponente A und/oder die Komponente B als Lösung/en in Schritt a) bereitgestellt werden.

7. Verfahren gemäss Anspruch 4 oder 5, wobei die Komponenten A und B als Feststoffe in Schritt a) bereitgestellt werden.

8. Verwendung von Zinnoctoat als Katalysator für die Trimerisierungsreaktion von OCN-Gruppen zu einem Triazinring in einem Cyanatester-basierten Klebstoff, insbesondere gemäss Anspruch 3, wobei das Zinnoctoat an einem Träger, insbesondere einer pyrogenen Kieselsäure, reversibel zurückgehalten ist.

9. Verwendung eines Cyanatester-basierten Klebstoffes gemäss einem der Ansprüche 1 bis 3 für die Beschichtung eines Substrats.

10. Substrat mit einer Beschichtung mit einem Cyanatester-basierten Klebstoff gemäss einem der Ansprüche 1 bis 3.

11. Verwendung eines Substrats beschichtet mit einem Cyanatester-basierten Klebstoff, insbesondere gemäss einem der Ansprüche 1 bis 3, für die Isolierung eines Bauteils im Bereich Automobilbau, Hausbau, Schiffsbau, Ofenbau, Hochofenbau, Chemiereaktoren, Flugzeugbau/-industrie, Luft- und Raumfahrt, Blockheizkraftwerke, Zementofen, Kehrricht-/Abfallverbrennungsofen.

12. Verfahren zur Beschichtung eines Bauteils mit einem Cyanatester-basierten Klebstoff gemäss einem der Ansprüche 1 bis 3 umfassend die Schritte
a) Aufbringen eines Cyanatester-basierten Klebstoffes auf ein Substrat,
b) Aufbringen des Bauteils auf den auf das Substrat aufgebrachten Cyanatester-basierten Klebstoff,
c) Vernetzen des Cyanatester-basierten Klebstoffes bei einer Temperatur von > 100°C, bevorzugt bei 220 °C, für 1 bis 30 min, bevorzugt 5 bis 10 min.

13. Verfahren gemäss Anspruch 12, wobei das Vernetzen unter Ausschluss von Luftfeuchtigkeit erfolgt.

14. Verfahren gemäss einem der Ansprüche 12 oder 13, wobei das Substrat und/oder das Bauteil vorbehandelt sind, insbesondere mittels chemischer und/oder physikalischer Reinigung, insbesondere Corona-Vorbehandlung oder Plasmabehandlung.

15. System umfassend
(i) mindestens einen Cyanatester aufweisend mindestens zwei OCN-Gruppen,
(ii) mindestens zwei unterschiedliche Katalysatoren für die Trimerisierungsreaktion von OCN-Gruppen zu einem Triazinring, welche an einem Träger, insbesondere einer pyrogenen Kieselsäure, reversibel zurückgehalten sind,
(iii)optional Anwendungshinweise, insbesondere für ein Verfahren nach einem der Ansprüche 12 bis 14.
